# FASCICULE DE BREVET EUROPEEN

(11) **EP 3 178 343 B1**
(45) Date de publication et mention de la délivrance du brevet: **26.06.2019**
(21) Numéro de dépôt: 15198534.8
(22) Date de dépôt: 08.12.2015
(51) Int. Cl.: A44C 5/00, A61M 35/00

(54) **BRACELET**
ARMBAND
BRACELET

(43) Date de publication de la demande: 14.06.2017
(73) Titulaire: The Swatch Group Research and Development Ltd., 2074 Marin (CH)
(72) Inventeur: François, Nicolas, 2000 Neuchâtel (CH)
(74) Mandataire: ICB SA

(56) Documents cités:
- CN-U- 203 234 155
- KR-U- 20080 003 342

## Description

### Domaine de l'invention

L'invention se rapporte à un bracelet, en particulier un bracelet pour montre, comprenant au moins une partie à base d'un matériau organique et destinée à être mise en contact avec la peau d'un utilisateur, ledit matériau organique contenant au moins un agent fonctionnel, par exemple un agent antibactérien.

La présente invention concerne également des procédés de fabrication dudit bracelet.

### Arrière-plan de l'invention

De tels bracelets comprenant un agent antibactérien sont connus et sont par exemple décrits dans le document DE 2031511 9U. Selon ce document, le bracelet de montre en cuir comprend une bande intérieure en contact avec la peau du porteur réalisée dans un cuir traité pour présenter des propriétés antibactériennes. Ce bracelet présente l'avantage de ne pas développer de mauvaises odeurs, notamment en cas de transpiration.

On connait aussi des bracelets réalisés en matières plastiques et comprenant des principes actifs utilisés sous forme de microcapsules enrobées dans le matériau du bracelet. Dans la publication KR 20080003342U, l'agent fonctionnel est un parfum, le bracelet contenant également une vitamine pour ses effets bénéfiques sur la peau. Dans la publication FR 2 842 107, l'agent fonctionnel peut être un parfum, ou une dose médicamenteuse. La vitamine ou le médicament sont libérés par frottement du bracelet sur la peau et diffuseront dans l'organisme pour traiter le porteur du bracelet.

Toutefois, certains principes actifs incorporés dans le bracelet, tels que les agents antibactériens ou les parfums, peuvent devenir irritants pour la peau, surtout en cas de contact prolongé du bracelet sur la peau, et plus particulièrement dans des conditions chaudes et humides. En effet, de telles conditions favorisent une macération à l'interface de la peau et du bracelet, ce qui crée pour le porteur de la montre, une sensation très inconfortable. Cumulé aux frottements du bracelet contre la peau, le port d'un bracelet dans lequel sont incorporés des principes actifs peut provoquer à plus ou moins long terme des irritations de la peau. Le port du bracelet devient alors inconfortable voire impossible en cas de lésions importantes de la peau.

### Résumé de l'invention

L'invention a notamment pour objectif de pallier les différents inconvénients des bracelets connus.

Plus précisément, un objectif de l'invention est de fournir un bracelet qui, bien qu'intégrant au moins un agent fonctionnel, permet de supprimer les risques d'irritation de la peau afin d'apporter un plus grand confort au porter.

A cet effet, la présente invention concerne un bracelet selon la revendication 1.

Ainsi, lorsque le bracelet selon l'invention est porté, le bracelet diffuse dans la peau de l'utilisateur au moins un agent cosmétique de la peau, agissant sur la peau au niveau du bracelet de manière à empêcher toute irritation de la peau. Le confort au porter est donc grandement amélioré.

La présente invention concerne également un procédé de fabrication d'un bracelet tel que défini ci-dessus, en particulier un bracelet pour montre, comprenant au moins une partie à base d'un matériau organique et destinée à être mise en contact avec la peau d'un utilisateur, ledit matériau organique contenant au moins un agent fonctionnel destiné à exercer une fonction thérapeutique ou non thérapeutique et choisi parmi le groupe comprenant les agents antibactériens, les parfums, les aents à effet froid, et leurs mélanges, et au moins un agent cosmétique de la peau choisi parmi le groupe comprenant un agent réparateur, un agent anti-irritant, et leurs mélanges, et agencé pour pouvoir se trouver au contact de la peau de l'utilisateur lors du port du bracelet, ledit agent cosmétique de la peau agissant sur la peau de l'utilisateur au niveau de bracelet afin de préserver son intégrité pour supprimer les risques d'irritation de la peau provoqués par l'agent fonctionnel, ledit procédé comprenant les étapes de :
a) préparer au moins un agent cosmétique de la peau sous une forme encapsulée agencée pour libérer ledit agent cosmétique par frottement sur la peau
b) réaliser une formulation à base dudit matériau organique
c) ajouter à la formulation obtenue à l'étape b) au moins un agent cosmétique de la peau sous la forme encapsulée obtenu à l'étape a) et au moins un agent fonctionnel
d) réaliser le bracelet à partir de la formulation obtenue à l'étape c).

La présente invention concerne également un procédé de fabrication d'un bracelet tel que défini ci-dessus, en particulier un bracelet pour montre, comprenant au moins une partie à base d'un matériau organique et destinée à être mise en contact avec la peau d'un utilisateur, ledit matériau organique contenant au moins un agent fonctionnel destiné à exercer une fonction thérapeutique ou non thérapeutique et choisi parmi le groupe comprenant les agents antibactériens, les parfums, les agents à effet froid, et leurs mélanges, et au moins un agent cosmétique de la peau choisi parmi le groupe comprenant un agent réparateur, un agent anti-irritant, et leurs mélanges, et agencé pour pouvoir se trouver au contact de la peau de l'utilisateur lors du port du bracelet, ledit agent cosmétique de la peau agissant sur la peau de l'utilisateur au niveau de bracelet afin de préserver son intégrité pour supprimer les risques d'irritation de la peau provoqués par l'agent fonctionnel, ledit procédé comprenant les étapes de :
a') se munir d'un élément destiné à former la partie à base du matériau organique du bracelet, ledit matériau organique étant sans agent cosmétique de la peau, et éventuellement sans agent fonctionnel
b') incorporer au moins un agent cosmétique de la peau, et éventuellement au moins un agent fonctionnel si ce dernier n'est pas déjà présent, dans la partie à base du matériau organique du bracelet selon l'étape a') par un procédé d'imprégnation
c') réaliser le bracelet, ladite étape c') pouvant s'effectuer entre l'étape a') et l'étape b') ou après l'étape b').

### Description détaillée des modes de réalisation préférés

La présente invention concerne un bracelet, en particulier un bracelet pour montre. Ce bracelet comprend au moins une partie à base d'un matériau organique et destinée à être mise en contact avec la peau d'un utilisateur. Cette partie peut constituer uniquement une ou plusieurs zones du bracelet ou la totalité du bracelet.

Dans la présente description, le terme « matériau organique » désigne de préférence le cuir, les élastomères, les élastomères thermoplastiques, ou tout autre matériau organique utilisé pour réaliser un bracelet, et notamment un bracelet de montre.

En ce qui concerne les élastomères, ceux-ci sont avantageusement des élastomères vulcanisables de type caoutchouc. Ils peuvent être réalisés par exemple à partir d'un élastomère fluoro-carboné, connu pour avoir un toucher agréable, ou d'un mélange d'élastomères fluoro-carbonés type copolymère de tétrafluoroéthylène/propylène d'un poids moléculaire de 70 000 à 250 000 ou de tétrafluoroéthylène/perfluorométhyl vinyl éther d'un poids moléculaire de 100 000 à 300 000 ou encore des terpolymères de tétrafluoroéthylène/propylène/fluorure de vinylidène d'un poids moléculaire de 100 000 à 250 000. De tels élastomères sont disponibles sur le marché. L'élastomère de type caoutchouc peut être mélangé à d'autres élastomères du type nitrile (NBR), nitrile hydrogéné (HNBR), ou silicone (VMQ, FMVQ,...).

L'élastomère thermoplastique peut être un élastomère thermoplastique de type polyuréthane ou un copolymère d'éthylène vinyl acétate, ou un copolymère bloc amide. De tels élastomères thermoplastiques sont disponibles sur le marché.

Le matériau organique contient au moins un agent fonctionnel choisi parmi le groupe comprenant les agents antibactériens, les parfums, les agents à effet froid, et leurs mélanges.

Dans la présente description, le terme « agent fonctionnel » désigne une substance incorporée dans le bracelet pour y exercer une certaine fonction qui peut être aussi bien thérapeutique que non thérapeutique. Ainsi, l'agent fonctionnel présent dans le bracelet peut être par exemple un médicament, un parfum, un agent antibactérien, un agent à effet froid tel que le menthyl lactate, et leurs mélanges, ou tout autre agent fonctionnel approprié pour conférer au bracelet la propriété recherchée. Selon sa fonction, l'agent fonctionnel peut être agencé pour pouvoir se trouver au contact de la peau de l'utilisateur lors du port du bracelet. De préférence, l'agent fonctionnel présent dans le bracelet est un agent antibactérien pouvant être choisi par exemple parmi le groupe comprenant la pyrithione de zinc, le phosphate d'argent, les sels d'aluminium, tel que le chlorhydrate d'aluminium, et des microsphérites absorbantes. Un tel agent antibactérien permet d'empêcher le développement de mauvaises odeurs dues essentiellement à la transpiration du porteur du bracelet. Le chlorhydrate d'aluminium a pour avantage d'être également un agent antitranspirant. Il est bien sûr possible de combiner différents agents fonctionnels, tels qu'un parfum et un agent antibactérien.

Lorsque le bracelet est en cuir, tous les éléments constitutifs du bracelet peuvent être traités avec un agent fonctionnel, tel qu'un agent antibactérien. Ainsi, le dessus du bracelet, la doublure, les fils de couture, les éléments intérieurs tels que le rembourrage ou les indéchirables peuvent être également traités avec un agent antibactérien.

Conformément à la présente invention, le matériau organique de la partie de bracelet destinée à être mise en contact avec la peau de l'utilisateur contient également au moins un agent cosmétique de la peau choisi parmi le groupe comprenant un agent réparateur, un agent anti-irritant, et leurs mélanges et agencé pour pouvoir se trouver au contact de la peau de l'utilisateur lors du port du bracelet. Dans la présente description, le terme « agent cosmétique de la peau » désigne un agent de soin cosmétique de la peau, c'est-à-dire un principe actif agissant au niveau de la peau pour la protéger, l'entretenir et/ou la réparer afin de préserver son intégrité. L'agent cosmétique de la peau est différent de l'agent fonctionnel.

D'une manière avantageuse, l'agent cosmétique de la peau peut être un agent réparateur qui a pour but d'agir sur la peau à long terme. Un tel agent réparateur peut être une céramide ou l'acide hyaluronique.

Selon un autre aspect avantageux de l'invention, l'agent cosmétique de la peau peut être un agent anti-irritant, qui a pour but d'agir sur la peau instantanément en calmant toute irritation. L'agent anti-irritant peut être de l'acétyl tetrapeptide-15 dispersé dans le mannitol et le citrate de sodium ou un extrait d'avoine dans une dispersion d'eau et de glycérine.

De préférence, un agent réparateur est utilisé en combinaison avec un agent anti-irritant.

D'une manière particulièrement avantageuse, l'agent cosmétique de la peau peut être sous une forme encapsulée agencée pour libérer ledit agent cosmétique par frottement sur la peau lorsque le bracelet est porté par l'utilisateur. Les techniques d'encapsulation de principe actif sont connues de l'homme du métier et ne nécessitent pas de description détaillée. Il est toutefois utile de préciser que l'agent cosmétique de la peau est encapsulé dans une résine suffisamment résistante pour résister au processus de mise en forme. Une telle résine est par exemple de la mélamine.

Le bracelet selon l'invention peut être obtenu selon différents procédés.

Selon une première variante, le procédé de l'invention comprend les étapes de :
a) préparer au moins un agent cosmétique de la peau sous une forme encapsulée agencée pour libérer ledit agent cosmétique par frottement sur la peau
b) réaliser une formulation à base dudit matériau organique
c) ajouter à la formulation obtenue à l'étape b) au moins un agent cosmétique de la peau sous la forme encapsulée obtenu à l'étape a) et au moins un agent fonctionnel
d) réaliser le bracelet à partir de la formulation obtenue à l'étape c).

Cette première variante s'applique plus particulièrement lorsque le matériau organique est choisi parmi les élastomères vulcanisables de type caoutchouc et les élastomères thermoplastiques.

Au moins un agent cosmétique de la peau est préparé selon les techniques d'encapsulation de principe actif connues. La taille des microcapsules est par exemple de 50 µm.

La formulation à base dudit matériau organique est une formulation traditionnellement utilisée pour réaliser des bracelets, et notamment des bracelets de montre, en élastomères de type caoutchouc ou de type thermoplastiques.

Par exemple, pour des élastomères vulcanisables de type caoutchouc, cette formulation peut comprendre d'une manière connue :
- l'élastomère ou un mélange d'élastomères, introduit pour 100parts de la formulation,
- une charge pour 20 à 40 parts, ladite charge étant par exemple de la silice, du kaolin, du TiO₂ si le bracelet est de couleur claire, ou du noir de carbone si le bracelet est de couleur foncée ou noire,
- un accélérateur de vulcanisation, tel qu'un oxyde de manganèse, pour 2 à 4 parts,
- un agent de vulcanisation, tel que des peroxydes, pour 4 à 6 parts,
- des additifs tels que de la cire de carnauba, pour 3 à 7 parts
- des additifs pour le système colorant, pour 0.5 à 2 parts.
Il est bien évident que toute autre formulation appropriée peut être utilisée.

Une fois la formulation préparée, au moins un agent cosmétique de la peau sous la forme micro-encapsulée et au moins un agent fonctionnel sont ajoutés à ladite formulation lors d'une étape de mélangeage. Par exemple, la teneur en agent cosmétique est comprise entre 1% et 40% en poids, de préférence entre 2% et 20% en poids, par rapport au poids total de la formulation, et la teneur en agent fonctionnel est comprise entre 0.1% et 1% en poids, et de préférence entre 0.2% et 0.4% en poids, par rapport au poids total de la formulation.

Le bracelet est ensuite réalisé par injection ou compression de la formulation obtenue comprenant l'agent cosmétique de la peau et au moins un agent fonctionnel. Ces techniques de fabrication sont connues en soi et ne nécessitent pas de description détaillée. L'avantage de ce procédé est de permettre une meilleure intégration de l'agent cosmétique de la peau et de l'agent fonctionnel dans le matériau organique, et en particulier pour les élastomères vulcanisables du fait de la vulcanisation qui va permettre de limiter les phénomènes de migration.

Selon une deuxième variante, le procédé de l'invention comprend les étapes de :
a') se munir d'un élément destiné à former la partie à base du matériau organique du bracelet, ledit matériau organique étant sans agent cosmétique de la peau, et éventuellement sans agent fonctionnel
b') incorporer au moins un agent cosmétique de la peau, et éventuellement au moins un agent fonctionnel si ce dernier n'est pas déjà présent, dans la partie à base du matériau organique du bracelet selon l'étape a') par un procédé d'imprégnation
c') réaliser le bracelet, ladite étape c') pouvant s'effectuer entre l'étape a') et l'étape b') ou après l'étape b').

Selon un premier mode de réalisation, le procédé d'imprégnation est réalisé par simple imprégnation. Ce mode de réalisation peut s'appliquer tout particulièrement lorsque l'élément destiné à former la partie à base du matériau organique est réalisé en cuir. De préférence, cet élément est la doublure du bracelet qui d'une manière avantageuse a déjà été traitée avec au moins un agent fonctionnel, tel qu'un agent antibactérien.

Selon ce premier mode de réalisation, l'étape a') est une étape de teinture ou une étape de nourriture du cuir, et plus particulièrement de la doublure du bracelet contenant déjà l'agent antibactérien, et l'étape d'imprégnation b') est réalisée conjointement à l'étape a'), au moins un agent cosmétique de la peau ayant été préalablement préparé sous une forme encapsulée ou une émulsion. De préférence, l'agent cosmétique de la peau est encapsulé dans un polymère afin de résister aux conditions de température et de pH imposées par l'étape de teinture ou de nourriture du cuir. La mélamine peut être utilisée par exemple.

Selon un deuxième mode de réalisation, le procédé d'imprégnation est un procédé d'imprégnation par fluide supercritique. Un tel procédé d'imprégnation peut comprendre les étapes suivantes :
- dissoudre au moins un agent cosmétique de la peau et au moins un agent fonctionnel si ce dernier n'est pas déjà présent dans le bracelet, dans un liquide support pour former une solution ; l'agent fonctionnel est utilisé à des teneurs de 0.5% à 10% en poids, et de préférence à une teneur de 2.5% en poids si l'agent fonctionnel est un agent antibactérien, et l'agent cosmétique de la peau est de préférence préparé sous la forme d'une émulsion (glycérine-eau) comprenant de préférence entre 4% et 10% en poids d'agent cosmétique.
- placer à pression ambiante l'élément destiné à former la partie à base du matériau organique dans une enceinte pouvant être mise sous pression ;
- fermer hermétiquement l'enceinte ;
- imprégner l'élément destiné à former la partie à base du matériau organique de la solution au moyen d'un fluide dans des conditions supercritiques ou proche de conditions supercritiques dans l'enceinte à une pression comprise entre 3 MPa et 6 MPa, à une température comprise entre 25°C et 65°C, de préférence égale à 35°C, pendant une durée comprise entre 1 min et 12 min, de préférence égale à 10 min ;
   - relâcher la pression dans l'enceinte pour que le liquide support diffuse à l'extérieur de l'élément destiné à former la partie à base du matériau organique et pour piéger l'agent cosmétique de la peau et éventuellement l'agent fonctionnel à l'intérieur de l'élément destiné à former la partie à base du matériau organique.

D'une manière avantageuse, le fluide supercritique est le dioxyde de carbone et le liquide support est choisi parmi l'hexane, l'isopropanol ou encore le d-limonène. De préférence, l'imprégnation est réalisée sous flux constant.

D'une manière avantageuse, il est ensuite prévu une étape d'élimination des résidus qui peut être réalisée par lavage à l'eau claire ou par essuyage de l'élément destiné à former la partie à base du matériau organique imprégné.

Lorsque l'élément destiné à former la partie à base du matériau organique du bracelet est choisi parmi le cuir, les élastomères, et les élastomères thermoplastiques, l'étape b') d'imprégnation par fluide supercritique peut être réalisée après l'étape c'), c'est-à-dire sur un bracelet déjà réalisé. Plus particulièrement, lorsque le matériau organique est un élastomère ou un élastomère thermoplastique, le procédé d'imprégnation en agent cosmétique de la peau et en agent fonctionnel par fluide supercritique est mis en oeuvre sur le bracelet déjà réalisé par injection ou par compression à partir d'une formulation élastomère sans agent cosmétique de la peau ni agent fonctionnel. Lorsque le matériau organique est du cuir, le procédé d'imprégnation en agent cosmétique de la peau par fluide supercritique est mis en oeuvre sur le bracelet en cuir à l'état fini et déjà traité avec un agent fonctionnel tel qu'un agent antibactérien.

Selon une autre possibilité, lorsque l'élément destiné à former la partie à base du matériau organique est du cuir, l'étape a') est une étape de découpe du cuir, l'étape b') d'imprégnation par fluide supercritique étant réalisée à proximité de l'étape a') et avant l'étape c'). Plus spécifiquement, l'étape b') d'imprégnation par fluide supercritique est réalisée après l'étape de découpe du cuir. Puis le bracelet en cuir est terminé.

Le bracelet selon l'invention permet de présenter les propriétés apportées par au moins un agent fonctionnel, notamment l'agent antibactérien, tout en préservant la peau de l'utilisateur, grâce à la diffusion d'au moins un agent cosmétique de la peau au niveau du contact entre le bracelet et la peau du porteur du bracelet. Ainsi, le confort au porter est grandement amélioré.

Bien entendu, la présente invention ne se limite pas aux exemples illustrés et est susceptible de diverses variantes et modifications qui apparaîtront de manière évidente à l'homme du métier.

## Revendications

1. Bracelet, en particulier bracelet pour montre, comprenant au moins une partie à base d'un matériau organique et destinée à être mise en contact avec la peau d'un utilisateur, ledit matériau organique contenant au moins un agent fonctionnel destiné à exercer une fonction thérapeutique ou non thérapeutique, et choisi parmi le groupe comprenant les agents antibactériens, les parfums, les agents à effet froid, et leurs mélanges, **caractérisé en ce que** ledit matériau organique contient également au moins un agent cosmétique de la peau choisi parmi le groupe comprenant un agent réparateur, un agent anti-irritant, et leurs mélanges, et agencé pour pouvoir se trouver au contact de la peau de l'utilisateur lors du port du bracelet, ledit agent cosmétique de la peau agissant sur la peau de l'utilisateur au niveau du bracelet afin de préserver son intégrité pour supprimer les risques d'irritation de la peau provoqués par l'agent fonctionnel.

2. Bracelet selon la revendication 1, **caractérisé en ce que** le matériau organique est choisi parmi le cuir, les élastomères, et les élastomères thermoplastiques.

3. Bracelet selon l'une des revendications précédentes, **caractérisé en ce que** l'agent antibactérien est choisi parmi le groupe comprenant la pyrithione de zinc, le phosphate d'argent, et les sels d'aluminium.

4. Bracelet selon l'une des revendications précédentes, **caractérisé en ce que** l'agent réparateur est choisi parmi le groupe comprenant les céramides et l'acide hyaluronique.

5. Bracelet selon l'une des revendications 1 à 3, **caractérisé en ce que** l'agent anti-irritant est choisi parmi le groupe comprenant l'acetyl tetrapeptide-15 et l'extrait d'avoine.

6. Bracelet selon l'une des revendications précédentes, **caractérisé en ce que** l'agent cosmétique de la peau est sous une forme encapsulée agencée pour libérer ledit agent cosmétique par frottement sur la peau.

7. Procédé de fabrication d'un bracelet selon l'une des revendications 1 à 6, en particulier un bracelet pour montre, comprenant au moins une partie à base d'un matériau organique et destinée à être mise en contact avec la peau d'un utilisateur, ledit matériau organique contenant au moins un agent fonctionnel destiné à exercer une fonction thérapeutique ou non thérapeutique et choisi parmi le groupe comprenant les agents antibactériens, les parfums, les agents à effet froid, et leurs mélanges, et au moins un agent cosmétique de la peau choisi parmi le groupe comprenant un agent réparateur, un agent anti-irritant, et leurs mélanges, et agencé pour pouvoir se trouver au contact de la peau de l'utilisateur lors du port du bracelet, ledit agent cosmétique de la peau agissant sur la peau de l'utilisateur au niveau du bracelet afin de préserver son intégrité pour supprimer les risques d'irritation de la peau provoqués par l'agent fonctionnel, **caractérisé en ce que** ledit procédé comprend les étapes de :
a) préparer au moins un agent cosmétique de la peau sous une forme encapsulée agencée pour libérer ledit agent cosmétique par frottement sur la peau
b) réaliser une formulation à base dudit matériau organique
c) ajouter à la formulation obtenue à l'étape b) au moins un agent cosmétique de la peau sous la forme encapsulée obtenu à l'étape a) et au moins un agent fonctionnel
d) réaliser le bracelet à partir de la formulation obtenue à l'étape c).

8. Procédé selon la revendication 7, **caractérisé en ce que** le matériau organique est choisi parmi les élastomères et les élastomères thermoplastiques.

9. Procédé de fabrication d'un bracelet selon l'une des revendications 1 à 6, en particulier un bracelet pour montre, comprenant au moins une partie à base d'un matériau organique et destinée à être mise en contact avec la peau d'un utilisateur, ledit matériau organique contenant au moins un agent fonctionnel destiné à exercer une fonction thérapeutique ou non thérapeutique et choisi parmi le groupe comprenant les agents antibactériens, les parfums, les agents à effet froid, et leurs mélanges, et au moins un agent cosmétique de la peau choisi parmi le groupe comprenant un agent réparateur, un agent anti-irritant, et leurs mélanges, et agencé pour pouvoir se trouver au contact de la peau de l'utilisateur lors du port du bracelet, ledit agent cosmétique de la peau agissant sur la peau de l'utilisateur au niveau du bracelet afin de préserver son intégrité pour supprimer les risques d'irritation de la peau provoqués par l'agent fonctionnel, **caractérisé en ce que** ledit procédé comprend les étapes de :
a') se munir d'un élément destiné à former la partie à base du matériau organique du bracelet, ledit matériau organique étant sans agent cosmétique de la peau, et éventuellement sans agent fonctionnel
b') incorporer au moins un agent cosmétique de la peau, et éventuellement au moins un agent fonctionnel si ce dernier n'est pas déjà présent, dans la partie à base du matériau organique du bracelet selon l'étape a') par un procédé d'imprégnation
c') réaliser le bracelet, ladite étape c') pouvant s'effectuer entre l'étape a') et l'étape b') ou après l'étape b').

10. Procédé selon la revendication 9, **caractérisé en ce que** le procédé d'imprégnation est un procédé d'imprégnation par fluide supercritique.

11. Procédé selon la revendication 10, **caractérisé en ce que** l'étape b') d'imprégnation par fluide supercritique est réalisée après l'étape c').

12. Procédé selon l'une des revendications 10 ou 11, **caractérisé en ce que** l'élément destiné à former la partie à base du matériau organique du bracelet est choisi parmi le cuir, les élastomères, et les élastomères thermoplastiques.

13. Procédé selon la revendication 10, dans lequel l'élément destiné à former la partie à base du matériau organique est du cuir, **caractérisé en ce que** l'étape a') est une étape de découpe du cuir, l'étape b') d'imprégnation par fluide supercritique étant réalisée à proximité de l'étape a') et avant l'étape c').

14. Procédé selon la revendication 9, dans lequel l'élément destiné à former la partie à base du matériau organique est du cuir, **caractérisé en ce que** l'étape a') est une étape de teinture ou une étape de nourriture du cuir, et **en ce que** l'étape d'imprégnation b') est réalisée conjointement à l'étape a'), l'agent cosmétique de la peau ayant été préalablement préparé sous une forme encapsulée.

15. Procédé selon l'une des revendications 7 à 14, **caractérisé en ce que** l'agent antibactérien est choisi parmi le groupe comprenant la pyrithione de zinc, le phosphate d'argent et les sels d'aluminium.

16. Procédé selon l'une des revendication 7 à 15, **caractérisé en ce que** l'agent réparateur est choisi parmi le groupe comprenant les céramides et l'acide hyaluronique.

17. Procédé selon l'une des revendications 7 à 16, **caractérisé en ce que** l'agent anti-irritant est choisi parmi le groupe comprenant l'acetyl tetrapeptide-15 et l'extrait d'avoine.

## Patentansprüche

1. Armband, insbesondere ein Uhrenarmband, umfassend mindestens einen Teil auf Basis eines organischen Materials, der dazu bestimmt ist, mit der Haut eines Benutzers in Kontakt gebracht zu werden, wobei das organische Material mindestens einen funktionalen Wirkstoff enthält, der dazu bestimmt ist, eine therapeutische oder nichttherapeutische Funktion auszuüben, und aus der Gruppe ausgewählt ist, die antibakterielle Wirkstoffe, Parfüms, Wirkstoffe mit Kältewirkung und deren Mischungen umfasst,
**dadurch gekennzeichnet, dass** das genannte organische Material auch mindestens einen kosmetischen Wirkstoff für die Haut enthält, der aus der Gruppe ausgewählt ist, die einen Repairwirkstoff, einen reizhemmenden Wirkstoff und deren Mischungen umfasst, und dafür ausgelegt ist, die Haut des Benutzers beim Tragen des Armbandes zu berühren, wobei der kosmetische Wirkstoff für die Haut des Benutzers im Bereich des Armbandes wirkt, um deren Unversehrtheit zu bewahren, um Gefahren von Reizungen der Haut, die durch den funktionalen Wirkstoff hervorgerufen werden, zu verhindern.

2. Armband nach Anspruch 1, **dadurch gekennzeichnet, dass** das organische Material aus Leder, Elastomeren und thermoplastischen Elastomeren ausgewählt ist.

3. Armband nach einem der vorhergehenden Ansprüche, **dadurch gekennzeichnet, dass** der antibakterielle Wirkstoff aus der Gruppe ausgewählt ist, die Zink-Pyrithion, Silberphosphat und Aluminiumsalze umfasst.

4. Armband nach einem der vorhergehenden Ansprüche, **dadurch gekennzeichnet, dass** der Repairwirkstoff aus der Gruppe ausgewählt ist, die Ceramide und Hyaluronsäure umfasst.

5. Armband nach einem der Ansprüche 1 bis 3, **dadurch gekennzeichnet, dass** der reizhemmende Wirkstoff aus der Gruppe ausgewählt ist, die Acetyl-Tetrapeptid-15 und Haferextrakt umfasst.

6. Armband nach einem der vorhergehenden Ansprüche, **dadurch gekennzeichnet, dass** der kosmetische Wirkstoff für die Haut verkapselt vorliegt und dafür ausgelegt ist, den kosmetischen Wirkstoff durch Reibung auf der Haut freizusetzen.

7. Verfahren für die Herstellung eines Armbandes nach einem der Ansprüche 1 bis 6, insbesondere eines Uhrenarmbandes, umfassend mindestens einen Teil auf Basis eines organischen Materials, der dazu bestimmt ist, mit der Haut eines Benutzers in Kontakt gebracht zu werden, wobei das organische Material mindestens einen funktionalen Wirkstoff, der dazu bestimmt ist, eine therapeutische oder nichttherapeutische Funktion auszuüben, und aus der Gruppe ausgewählt ist, die antibakterielle Wirkstoffe, Parfüms, Wirkstoffe mit Kältewirkung und deren Mischungen umfasst, und mindestens einen kosmetischen Wirkstoff für die Haut, der aus der Gruppe ausgewählt ist, die einen Repairwirkstoff, einen reizhemmenden Wirkstoff und deren Mischungen umfasst, und dafür ausgelegt ist, die Haut des Benutzers beim Tragen des Armbandes zu berühren, umfasst, wobei der kosmetische Wirkstoff für die Haut des Benutzers im Bereich des Armbandes wirkt, um deren Unversehrtheit zu bewahren, um die Gefahren einer Reizung der Haut, die durch den funktionalen Wirkstoff hervorgerufen werden, zu verhindern, **dadurch gekennzeichnet, dass** das Verfahren die Schritte umfasst:
a) Bereitstellen mindestens eines kosmetischen Wirkstoffs für die Haut in verkapselter Form, die dafür ausgelegt ist, den kosmetischen Wirkstoff durch Reibung auf der Haut freizusetzen,
b) Herstellen einer Formulierung auf der Basis des organischen Materials,
c) Hinzufügen mindestens eines kosmetischen Wirkstoffs für die Haut in verkapselter Form, der im Schritt a) erhalten wird, und mindestens eines funktionalen Wirkstoffs zu der im Schritt b) erhaltenen Formulierung,
d) Herstellen des Armbandes anhand der im Schritt c) erhaltenen Formulierung.

8. Verfahren nach Anspruch 7, **dadurch gekennzeichnet, dass** das organische Material aus Elastomeren und thermoplastischen Elastomeren ausgewählt ist.

9. Verfahren für die Herstellung eines Armbandes nach einem der Ansprüche 1 bis 6, insbesondere eines Uhrenarmbandes, umfassend mindestens einen Teil auf Basis eines organischen Materials, der dazu bestimmt ist, mit der Haut eines Benutzers in Kontakt gebracht zu werden, wobei das organische Material mindestens einen funktionalen Wirkstoff, der dazu bestimmt ist, eine therapeutische oder nichttherapeutische Funktion auszuüben, und aus der Gruppe ausgewählt ist, die antibakterielle Wirkstoffe, Parfüms, Wirkstoffe mit Kältewirkung und deren Mischungen umfasst, und mindestens einen kosmetischen Wirkstoff für die Haut, der aus der Gruppe ausgewählt ist, die einen Repairwirkstoff, einen reizhemmenden Wirkstoff und deren Mischungen umfasst, und dafür ausgelegt ist, die Haut des Benutzers beim Tragen des Armbandes zu berühren, umfasst, wobei der kosmetische Wirkstoff für die Haut im Bereich des Armbandes auf die Haut des Benutzers wirkt, um deren Unversehrtheit zu bewahren, um die Gefahren einer Reizung der Haut, die durch den funktionalen Wirkstoff hervorgerufen werden, zu verhindern,
**dadurch gekennzeichnet, dass** das Verfahren die Schritte umfasst:
a') Bereitstellen eines Elements, das dazu bestimmt ist, den Teil auf Basis eines organischen Materials des Armbandes zu bilden, wobei das organische Material keinen kosmetischen Wirkstoff für die Haut und gegebenenfalls keinen funktionalen Wirkstoff enthält,
b') Zusetzen mindestens eines kosmetischen Wirkstoffs für die Haut und gegebenenfalls mindestens eines funktionalen Wirkstoffs, falls dieser Letztere nicht bereits vorhanden ist, in dem Teil auf Basis des organischen Materials des Armbandes gemäß Schritt a') durch ein Tränkungsverfahren,
c') Herstellen des Armbandes, wobei Schritt c') zwischen Schritt a') und Schritt b') oder nach Schritt b') ausführbar ist.

10. Verfahren nach Anspruch 9, **dadurch gekennzeichnet, dass** das Tränkungsverfahren ein Verfahren zum Tränken mit einem überkritischen Fluid ist.

11. Verfahren nach Anspruch 10, **dadurch gekennzeichnet, dass** der Schritt b') zum Tränken mit einem überkritisches Fluid nach Schritt c') ausgeführt wird.

12. Verfahren nach einem der Ansprüche 10 oder 11, **dadurch gekennzeichnet, dass** das Element, das dazu bestimmt ist, den Teil auf der Basis eines organischen Materials des Armbandes zu bilden, aus Leder, Elastomeren und thermoplastischen Elastomeren ausgewählt ist.

13. Verfahren nach Anspruch 10, wobei das Element, das dazu bestimmt ist, den Teil auf der Basis eines organischen Materials zu bilden, Leder ist, **dadurch gekennzeichnet, dass** Schritt a') ein Schritt zum Schneiden von Leder ist, wobei Schritt b') zum Tränken mit einem überkritischen Fluid nahe am Schritt a') und vor Schritt c') ausgeführt wird.

14. Verfahren nach Anspruch 9, wobei das Element, das dazu bestimmt ist, den Teil auf der Basis eines organischen Materials zu bilden, Leder ist, **dadurch gekennzeichnet, dass** Schritt a') ein Schritt zum Färben oder ein Schritt zur Fettung von Leder ist und dass der Schritt zum Tränken b') gemeinsam mit Schritt a') ausgeführt wird, wobei der kosmetische Wirkstoff für die Haut im Voraus verkapselt bereitgestellt worden ist.

15. Verfahren nach einem der Ansprüche 7 bis 14, **dadurch gekennzeichnet, dass** der antibakterielle Wirkstoff aus der Gruppe ausgewählt ist, die Zink-Pyrithion, Silberphosphat und Aluminiumsalze umfasst.

16. Verfahren nach einem der Ansprüche 7 bis 15, **dadurch gekennzeichnet, dass** der Repairwirkstoff aus der Gruppe ausgewählt ist, die Ceramid und Hyaluronsäure umfasst.

17. Verfahren nach einem der Ansprüche 7 bis 16, **dadurch gekennzeichnet, dass** der reizhemmende Wirkstoff aus der Gruppe ausgewählt ist, die Acetyl-Tetrapeptid-15 und Haferextrakt umfasst.

## Claims

1. Bracelet or strap, in particular a watch band, comprising at least one organic material based portion intended to be placed in contact with a user's skin, said organic material containing at least one functional agent intended to exert a therapeutic or non-therapeutic function, and chosen from the group comprising antibacterial agents, perfumes, cooling agents, and mixtures thereof, **characterized in that** said organic material also contains at least one cosmetic agent for skin chosen from the group comprising a repair agent, an anti-irritant agent and mixtures thereof, and arranged to be able to be in contact with the user's skin when the bracelet or strap is worn, said cosmetic agent for skin acting on the user's skin at the band so as to preserve the integrity thereof in order to eliminate the risks of irritation of the skin caused by the functional agent.

2. Bracelet or strap according to claim 1, **characterized in that** the organic material is chosen from the group comprising leather, elastomers, and thermoplastic elastomers.

3. Bracelet or strap according to any of the preceding claims, **characterized in that** the antibacterial agent is chosen from the group comprising zinc pyrithione, silver phosphate and aluminium salts.

4. Bracelet or strap according to any of the preceding claims, **characterized in that** the repair agent is chosen from the group comprising ceramides and hyaluronic acid.

5. Bracelet or strap according to any of claims 1 to 3, **characterized in that** the anti-irritant agent is chosen from the group comprising acetyl tetrapeptide-15 and oat extract.

6. Bracelet or strap according to any of the preceding claims, **characterized in that** the cosmetic agent for skin is in encapsulated form arranged to release said cosmetic agent through friction on the skin

7. Method for manufacture of a bracelet or strap according to any of claims 1 to 6, in particular a watch band, comprising at least one organic material based portion intended to be placed in contact with the user's skin, said organic material containing at least one functional agent intended to exert a therapeutic or non-therapeutic function, and chosen from the group comprising antibacterial agents, perfumes, cooling agents, and mixtures thereof, and at least one cosmetic agent for the skin chosen from the group comprising a repair agent, an anti-irritant agent and mixtures thereof, and arranged to be able to be in contact with the user's skin when the bracelet or strap is worn, said cosmetic agent for skin acting on the user's skin at the band so as to preserve the integrity thereof in order to eliminate the risks of irritation of the skin caused by the functional agent, **characterized in that** said method comprises the steps of:
a) preparing at least one cosmetic agent for skin in encapsulated form arranged to release said cosmetic agent through friction on the skin
b) producing a formulation using said organic material
c) adding to the formulation obtained in step b) at least one cosmetic agent for skin in the encapsulated form obtained in step a) and at least one functional agent
d) making the bracelet or strap from the formulation obtained in step c).

8. Method according to claim 7, **characterized in that** the organic material is chosen from elastomers and thermoplastic elastomers.

9. Method for manufacture of a bracelet or strap according to any of claims 1 to 6, in particular a watch band, comprising at least one organic material based portion intended to be placed in contact with the user's skin, said organic material containing at least one functional agent intended to exert a therapeutic or non-therapeutic function, and chosen from the group comprising antibacterial agents, perfumes, cooling agents, and mixtures thereof, and at least one cosmetic agent for the skin chosen from the group comprising a repair agent, an anti-irritant agent and mixtures thereof, and arranged to be able to be in contact with the user's skin when the bracelet or strap is worn, said cosmetic agent for skin acting on the user's skin at the band so as to preserve the integrity thereof in order to eliminate the risks of irritation of the skin caused by the functional agent, **characterized in that** said method comprises the steps of:
a') taking an element intended to form the organic material based portion of the bracelet or strap, said organic material having no cosmetic agent for skin, and optionally no functional agent
b') incorporating at least one cosmetic agent for skin, and optionally at least one functional agent if the latter is not already present, in the organic material based portion of the bracelet or strap in step a') by a method of impregnation
c') making the bracelet or strap, said step c') can be performed between step a') and step b') or after step b').

10. Method according to claim 9, **characterized in that** the impregnation method is a supercritical fluid impregnation method.

11. Method according to claim 10, **characterized in that** the supercritical fluid impregnation step b') is performed after step c').

12. Method according to either of claims 10 or 11, **characterized in that** the element intended to form the organic material based portion of the bracelet or strap is chosen from the group comprising leather, elastomers and thermoplastic elastomers.

13. Method according to claim 10, wherein the element intended to form the organic material based portion is leather, **characterized in that** step a') is a leather cutting step, and supercritical fluid impregnation step b') is performed close to step a') and before step c').

14. Method according to claim 9, wherein the element intended to form the organic material based portion is leather, **characterized in that** step a') is a dyeing step or a leather nourishing step, and **in that** the impregnation step b') is performed jointly with step a'), the cosmetic agent for skin having been pre-prepared in an encapsulated form.

15. Method according to any of claims 7 to 14, **characterized in that** the antibacterial agent is chosen from the group comprising zinc pyrithione, silver phosphate and aluminium salts.

16. Method according to any of claims 7 to 15, **characterized in that** the repair agent is chosen from the group comprising ceramides and hyaluronic acid.

17. Method according to any of claims 7 to 16, **characterized in that** the anti-irritant agent is chosen from the group comprising acetyl tetrapeptide-15 and oat extract.
